# EUROPEAN PATENT SPECIFICATION

(11) **EP 4 043 069 B1**
(45) Date of publication and mention of the grant of the patent: **15.10.2025**
(21) Application number: 22156700.1
(22) Date of filing: 15.02.2022
(51) Int. Cl.: A61N 1/375, A61B 8/08, A61M 25/10

(54) **ECHOGENIC DELIVERY SYSTEM FOR LEADLESS PACEMAKER**
ECHOGENES ZUFÜHRUNGSSYSTEM FÜR EINEN LEITUNGSLOSEN HERZSCHRITTMACHER
SYSTÈME DE DISTRIBUTION ÉCHOGÈNE POUR STIMULATEUR CARDIAQUE SANS FIL

(30) Priority: 15.02.2021 US 202163149577 P; 04.02.2022 US 202217650026
(43) Date of publication of application: 17.08.2022
(73) Proprietor: Medtronic, Inc., Minneapolis, Minnesota 55432 (US)
(72) Inventor: Schmidt, Elliot C., Mounds View, 55112 (US); Drake, Ronald A., Mounds View, 55112 (US)
(74) Representative: Zimmermann & Partner Patentanwälte mbB

(56) References cited:
- WO-A1-2020/018353
- DE-U1- 202019 104 557
- US-A1- 2007 156 197
- US-A1- 2017 136 231
- US-A1- 2018 256 902
- US-A1- 2021 001 085

## Description

### BACKGROUND

The leadless pacemaker, which is significantly smaller than a conventional pacemaker coupled to one or more transvenous leads, is a self-contained generator and electrode system implanted directly into the heart. The leadless pacemaker eliminates several complications associated with transvenous pacemakers and leads such as, for example, pocket infections, hematoma, lead dislodgment, and lead fracture. The leadless pacemaker also has cosmetic appeal because there is no chest incision or visible pacemaker pocket.

The leadless pacemaker device may be implanted via a femoral vein transcatheter approach, and requires no chest incision or subcutaneous generator pocket. The catheter system utilized to deploy the leadless pacemaker includes a distal end with a delivery cup housing the self-contained generator and electrode system, referred to herein as a pacing capsule. The delivery cup is maneuvered into the proper position, e.g., in the right ventricle, using a sonogram produced by an ultrasound imaging system, and the pacing capsule is then implanted using an arrangement of flexible tines extending from the body of the capsule.

DE 20 2019 104557 U1 relates to a catheter-based system for detecting/aligning the orientation of an intracardiac pacemaker during a pacemaker implantation procedure. US 2021/041085 A1 relates to a catheter for ultrasound-guided delivery.

### SUMMARY

The invention is defined by independent claim 1. The pacing capsule of the leadless pacemaker device should be securely implanted in a desired location, e.g., in the right ventricle of the heart, and sonograms formed by an ultrasonic probe provide a practitioner with guidance to maneuver the deployment catheter through a femoral vein and into the heart. The pacing capsule of the leadless pacemaker device is deployed and implanted using a delivery catheter including a distal end with a rigid, generally cylindrical isodiometric delivery cup. Prior to deployment, the pacing capsule, which also has a generally cylindrical shape, resides within the delivery cup such that a distal end of the pacing capsule is substantially aligned with a distal tip of the delivery cup. Since the pacing capsule and the delivery cup have substantially the same cylindrical shape, the pacing capsule and the delivery cup together form a monolithic structure that can make the distal tip of the delivery cup difficult to discern in a sonogram image used to monitor the position of the delivery cup during an implantation procedure. In addition, the pacing capsule can cast a shadow that partially or even fully obscures the distal tip of the delivery cup in the sonogram image. Either or both of the shadowing effect and the shape of the pacing capsule can reduce the effectiveness of the sonograms during the procedures in which the pacing capsule is deployed and implanted, and can hinder accurate placement of the pacing capsule at a desired location within the heart.

In general, the present disclosure is directed to a system including a catheter for use in the delivery and implantation of a leadless pacemaker. The catheter includes a distal end with a delivery cup having a first portion configured to releasably retain a pacing capsule of the leadless pacemaker. The delivery cup of the present disclosure includes a second portion having an echogenic structure that improves the quality of sonograms used to track the position of the delivery cup during implantation procedures.

For example, in some examples, the echogenic structure projects away from the delivery cup so that that the delivery cup is not in the shadow of the pacing capsule when the pacing capsule is viewed from a wide variety of viewing angles. The echogenic structure ensures that contact between the delivery cup and tissue is clearly visible, and provides unobstructed sonogram images with improved clarity showing both the location of the distal tip of the delivery cup and the cardiac anatomy/tissue of a patient. The unobstructed sonogram images provide improved confirmation of the location of the distal end of the delivery cup, as well as the implantation status of the pacing capsule in cardiac tissue of the patient. Additionally, the shape of the distal tip improves the ability to determine alignment of the delivery cup with the anatomy displayed in a two-dimensional sonogram.

In one aspect, the present disclosure is directed to a catheter for delivery of a leadless pacemaker. The catheter includes an elongate flexible tubular body with a proximal end and a distal end, wherein the distal end of the tubular body has a delivery cup configured to releasably retain a pacing capsule of the leadless pacemaker. The delivery cup includes an echogenic structure.

In another aspect, the present disclosure is directed to a system for delivery of a leadless pacemaker. The system includes a pacing capsule and a catheter configured to deliver the pacing capsule to a target tissue. The catheter includes an elongate flexible tubular body with a distal end having a delivery cup configured to retain the pacing capsule. The delivery cup includes at least one echogenic structure.

In another aspect, the present disclosure is directed to a method for implanting a leadless pacemaker in a target tissue. The method includes inserting into a femoral vein of a patient a system for delivery of the leadless pacemaker. The system includes a pacing capsule and a catheter. The catheter includes an elongate flexible tubular body with a distal end having a delivery cup configured to retain the pacing capsule, wherein the delivery cup includes at least one echogenic structure. The method further includes monitoring the location of the at least one echogenic structure with an ultrasonic imager to form a sonogram; maneuvering the delivery cup as shown in the sonogram into a predetermined location in a heart of the patient; deploying the pacing capsule from the delivery cup to implant the pacing capsule into the predetermined location in the heart; and removing the catheter from the femoral vein.

The details of one or more examples are set forth in the accompanying drawings and the description below. Other features, objects, and advantages will be apparent from the description and drawings, and from the claims.

### BRIEF DESCRIPTION OF DRAWINGS

FIG. 1A is schematic perspective view of an example system for deploying a leadless pacemaker.
FIG. 1B is a schematic cross-sectional view of a delivery cup on a distal end of the catheter of the system of FIG. 1A.
FIG. 2 is a schematic cross-sectional view of an example of a delivery cup of the present disclosure that includes a tapered echogenic structure.
FIG. 3 is a schematic cross-sectional view of an example of a delivery cup of the present disclosure that includes a tapered echogenic structure with a hinge element.
FIG. 4 is a schematic cross-sectional view of an example of a delivery cup of the present disclosure that includes a tapered echogenic structure with a mechanical hinge element.
FIG. 5A is a schematic cross-sectional view of an example of a delivery cup of the present disclosure that includes an echogenic balloon.
FIG. 5B is a schematic cross-sectional view of an example of a delivery cup of the present disclosure that includes a plurality of echogenic balloons.
FIG. 6 is a flow chart of an illustrative example of a method for implanting a leadless pacemaker utilizing the echogenic delivery cups of the present disclosure.

Like symbols in the drawings indicate like elements.

### DETAILED DESCRIPTION

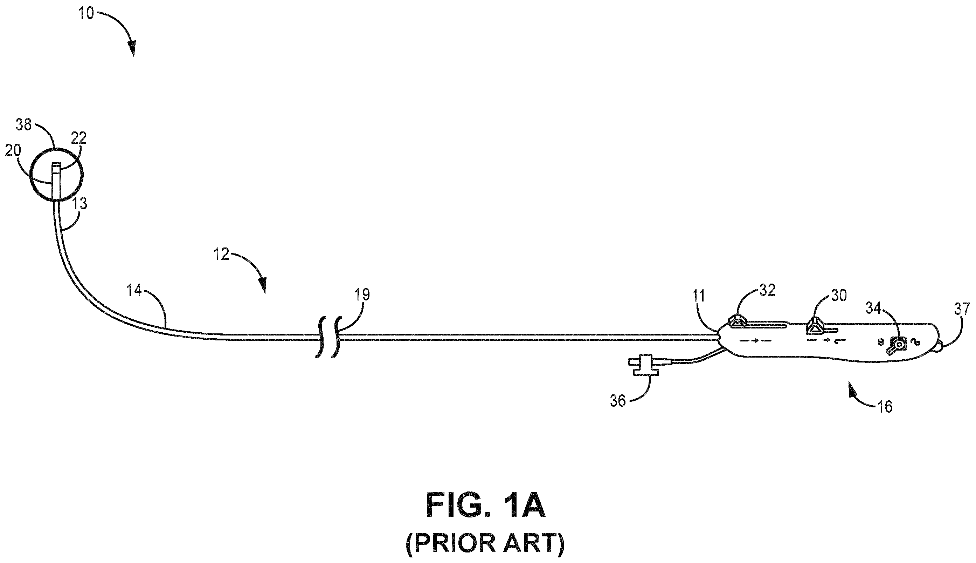

FIG. 1A is a schematic illustration (which is not to scale) of a system 10 for guiding and implanting a leadless pacemaker into a target tissue of a patient. The system 10 includes an elongate tubular catheter 12 having a body 14 with an elongate bore 19 that extends from a proximal end 11 to a distal end 13 thereof. In some examples, which are not intended to be limiting, the catheter body 14 has a length of about 100 centimeters (cm) to about 150 cm.

The proximal end 11 of the catheter body 14 is connected to a control handle 16 that can be used to deflect the catheter body 14 and deploy a pacing capsule 20. The pacing capsule 20 is retained at the distal end 13 of the catheter body 14 in a delivery cup 22. Suitable leadless pacemaker pacing capsules 20 include those available from Medtronic, Inc., Minneapolis, MN, under the trade designation MICRA, as well as those available from Abbot Laboratories, Abbot Park, IL, under the trade designation NANOSTIM. The pacing capsules 20 include a self-contained generator and electrode system that is implantable into cardiac tissue, and do not require leads or a subcutaneous pacemaker pocket like a transvenous pacemaker system.

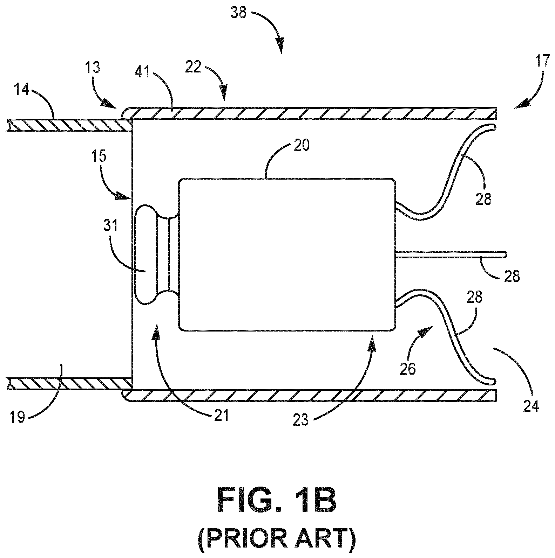

The catheter body 14 may be placed in a femoral vein of a patient and moved through the venous system to place a distal end 23 of the delivery cup 22 at a predetermined location in the heart, such as the right ventricle of the heart. In some examples, the catheter body 14 may be deflected using an optional curve deflection control 30 on the handle 16. The location of the catheter body 14 and a distal region 38 of the delivery cup 22 is monitored with an ultrasonic imaging system, and a sonogram image of the catheter body 14 and the delivery cup 22 is used to precisely position the delivery cup 22 in the heart. During placement procedures a proximal portion of the pacing capsule 20 remains tethered via a mechanical tether (not shown in FIG. 1) bound to a tether pin 37.

Once the delivery cup 22 is positioned at the proper location within the heart, the pacing capsule 20 is deployed from the delivery cup 22 using a deployment control 32 on the handle 16. The pacing capsule 20 can be implanted into the cardiac tissue using, for example, an arrangement of self-expanding metal tines, a screw-in metal helix, and combinations thereof, After the pacing capsule 20 is implanted in the tissue of the heart, a tether lock 34 on the handle 16 is released, and the catheter body 14 is withdrawn from the vascular system of the patient.

In some examples, the handle 16 may optionally include a fluid port 36, which can provide a fluid flush through the catheter body 14 using a fluid such as, for example, water, saline, and the like.

In some examples (not shown in FIG. 1A), the catheter 12 may optionally be placed within a rigid introducer including a tapered distal dilating tip to ease introduction of the catheter body 14 into the vasculature of the patient. For example, the dilating tip may be formed from an elastomeric material such as a silicone. In some examples, the introducer may optionally be coated with a lubricious hydrophilic coating.

Referring now to FIG. 1B, the distal region 38 of the catheter body 14 of the system 10 of FIG. 1A is shown in more detail. The distal end 13 of the catheter body 14 includes the generally cylindrical and isodiometric delivery cup 22, which includes a wall 41 configured to securely retain the generally cylindrical pacing capsule 20 of the leadless pacemaker as the catheter body 14 is maneuvered through the vasculature of the patient. The delivery cup 22 includes a first end 15 that is integral with or connected to the distal end 13 of the tubular body 14 of the catheter 12. A second distal end 17 of the delivery cup 22 includes an aperture 24 through which the pacing capsule 20 is deployed.

The pacing capsule 20 includes a first proximal end 21 retained in the delivery cup 22 proximal the first end 15 thereof. The first proximal end 21 of the delivery cup 22 includes a tether retention structure 31 configured to attach to a mechanical tether (not shown in FIG. 1B). A second distal end 23 of the delivery cup 22 is proximal the deployment aperture 24. The second end 23 of the pacing capsule 20 includes an implanting mechanism 26, which in the example of FIG. 1A includes an arrangement of a plurality of self-deploying metal tines 28. While retained in the delivery cup 22, the distal end 23 of the pacing capsule 20, as well as the tines 28, are substantially aligned near the distal end 17 of the delivery cup 22, and the tines 28 do not protrude from the aperture 24. The wall 41 of the delivery cup 22 maintains the orientation of the tines 28 until the pacing capsule 22 is deployed into a target tissue. When the pacing capsule 20 exits the delivery cup 22, the tines 28 spring back into a preformed hook-like shape as the tines 28 pierce into the target tissue.

Since the pacing capsule 20 and the delivery cup 22 have substantially the same cylindrical shape, the pacing capsule 20 and the delivery cup 22 together form a monolithic structure that can make the distal tip 17 of the delivery cup 22 difficult to discern in a sonogram image used to monitor the position of the distal catheter region 38 during implantation procedures. In addition, the pacing capsule 20 can cast a shadow that partially or even fully obscures the distal tip 17 of the delivery cup 22 in a sonogram image. Either or both of the shadowing effect and the shape of the pacing capsule 20 can reduce the effectiveness of the sonograms during the procedures in which the pacing capsule 20 is deployed and implanted from the delivery cup 22, and can hinder accurate placement of the pacing capsule 20 at a desired location within the right ventricle of the heart.

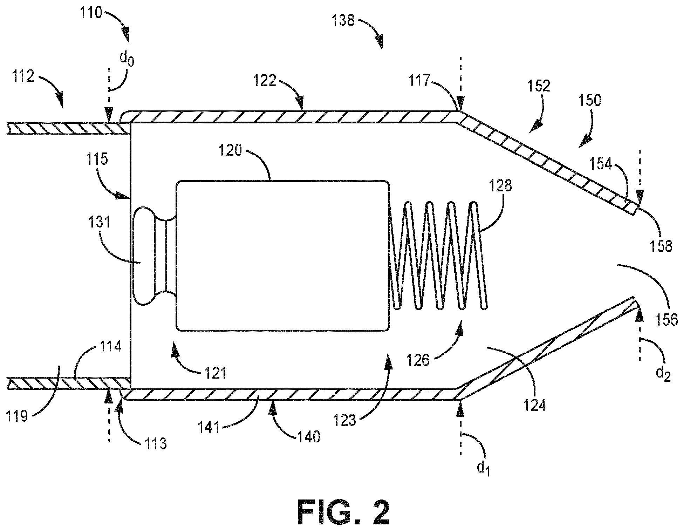

Referring now to FIG. 2, a distal region 138 of a catheter 112 of a leadless pacemaker deployment system 110 includes a tubular catheter body 114 with an elongate bore 119. The catheter body 114 can be made of any flexible material, including metals, polymeric materials, and the like. In some examples, the catheter 114 is formed by extrusion of a polymeric material including, but not limited to, polyethylene (PE), nylon, polypropylene (PP), polyether block amide (PEBA), polybutylene terephthalate (PBT), and combinations thereof. In other examples, the catheter body 114 can be formed by processes including, but not limited to, molding, three-dimensional (3D) printing, additive manufacturing, and the like. In various examples, the catheter body 114 can be formed from a single layer of polymeric material, or multiple layers of the same or different polymeric materials.

In some examples, which are not intended to be limiting, the catheter body 114 can have an outside diameter *dₒ* of about 0.100 inches (2.54 mm) to about 0.500 inches (12.7 mm), or typically about 0.200 inches (5.08 mm). In some examples, the catheter body 114 can optionally include a reinforcing or a catheter deflection material such as, for example, metal strands, ribbons, wires and the like (not shown in FIG. 2).

A distal end 113 of the catheter body 114 includes a delivery cup 122, which includes a generally cylindrical and isodiometric first portion 140 with a wall 141 configured to securely retain a generally cylindrical pacing capsule 120 of a leadless pacemaker as the catheter body 114 is maneuvered through patient vasculature. The first end 115 of the first portion 140 of the delivery cup 122 includes a first end 115 that is integral with or connected to the distal end 113 of the tubular body 114 of the catheter 112. A second distal end 117 of the first portion 140 of the delivery cup 122 includes an aperture 124.

In various examples, the first portion 140 of the delivery cup 122 may be made from a metal, a ceramic material, or a polymeric material that may be the same or different from the polymeric material used to form the tubular body 114. In some examples, the delivery cup 122 may be made from a high impedance acoustic material, which in the present application refers to materials having an acoustic impedance higher than the acoustic impedance of a target tissue into which the pacing capsule 120 is to be implanted. In some examples, the delivery cup may be made from a low impedance acoustic material, which refers herein to materials having an acoustic impedance lower than the acoustic impedance of the target tissue.

In various examples, the delivery cup 122 may be a portion of the tubular body 114, may be a separate structure press-fit into the tubular body 114, or may be a separate structure bonded to the tubular body 114 by an adhesive, ultrasonic welding, overmolding, and the like. Like the catheter body 114, the delivery cup 122 may be formed by a wide variety of manufacturing processes including, but not limited to, extrusion, molding, 3D printing, additive manufacturing, and the like.

The pacing capsule 120 includes a first end 121 retained in the first portion 140 of the delivery cup 122 proximal the first end 115 thereof, and a second distal end 123 proximal the aperture 124. The second end 123 of the pacing capsule 120 includes an implanting mechanism 126 with a screw-in helix tip 128. The implanting mechanism 126 is substantially aligned with the distal end 117 of the first portion 140 of the delivery cup 122 such that the mechanism 126 does not protrude from the aperture 124. In another example (not shown in FIG. 2), the implanting mechanism 126 could include the extended tines 128 shown in FIG. 1B that retract upon deployment of the pacing capsule 120 and assume a hook-like shape.

In FIG. 2 the delivery cup 122 includes a second portion 150 that forms a tapered echogenic structure 152 extending away from the distal end 117 of the first portion 140. In this application, the term echogenic structure refers to any structure extending from the distal end 117 of the first portion 140 of the delivery cup 122 that more effectively reflects or transmits ultrasound waves in the context of surrounding tissues to provide a more precise view of the location of the distal end 117. The echogenic structure 152 formed by the second portion 150 of the delivery cup 122 forms an interface with surrounding tissues that provides a more visible contrast difference on a sonogram, and makes the delivery cup 122 more readily identifiable for a practitioner maneuvering the catheter 112 and delivery cup 122 in a procedure in which the pacing capsule 120 is to be implanted in a target region of cardiac tissue. Since the echogenic structure 152 forms a more distinct contrast with surrounding cardiac tissue, the echogenic structure 152 can provide additional location information on the sonogram for the delivery cup 122. The echogenic structure 152 extends beyond the distal tip 117 of the first portion 140 of the delivery cup 122, and does not reside in the shadow of the pacing capsule 120 when viewed in a sonogram from a wide variety of viewing angles. This allows for unobstructed vision of both the echogenic structure 152 and the anatomy/tissue in the vicinity of the implantation site, which can ensure that contact between the delivery system 110 and the cardiac tissue is more clearly visible in a sonogram.

In the example of FIG. 2, the echogenic structure 152 includes a tapering conical protrusion 154 that forms a distal tip 158 and a deployment aperture 156 for the pacing capsule 120. As shown in the example of FIG. 2, a diameter d₁ of the aperture 124 is greater than the diameter d₂ of the deployment aperture 156 formed by the conical structure 154. The diameter d₂ of the deployment aperture 156 is also smaller than a diameter of the pacing capsule 120. In various examples, which are not intended to be limiting and are provided as an example, the diameter d₁ of the aperture 124 is about 0.100 inches (2.54 mm) to about 0.500 inches (12.7 mm), or typically about 0.275 inches (6.99 mm), and the diameter d₂ of the deployment aperture 156 is about 0.033 inches (0.838 mm) to about 0.165 inches (4.19 mm), or typically about 0.091 inches (2.31 mm).

In the example of FIG. 2, the tapered shape of the echogenic structure 152 provides orientation feedback to the user. The user looks for a sharp point formed by the distal tip 158 of the echogenic structure 152 to confirm that the delivery cup 122 is well oriented within an imaging plane. If the user sees a circular cross-section, the delivery cup 122 is oriented perpendicular to the imaging plane, and if the pacing capsule 120 is located between these two extremes (e.g. the pacing capsule 120 is crossing the imaging plane at 45 degrees), the shape of the distal tip 158 will appear on the sonogram as a shallow, foreshortened taper. These benefits are all gained without an increase in the overall diameter d₁ of the first portion 140 of the delivery cup 122, which can be an important design parameter, since the pacing capsule 120 and delivery cup 122 should be made as small as possible.

In some examples, the conical protrusion 154 is formed from a flexible material so that the distal aperture 156 can expand and allow deployment of the larger diameter pacing capsule 120 and the implanting mechanism 126. In some examples, the conical echogenic structure 152 is formed from an elastomeric polymeric material, which may be more flexible and compliant than the first portion 140 of the delivery cup 122, yet has structural rigidity sufficient such that the distal tip 158 can maintain contact with a target tissue such as the wall of the heart. In other examples, the delivery cup 122 and the conical echogenic structure 152 may be made from the same elastomeric polymeric material. In some example examples, which are not intended to be limiting, suitable elastomeric polymeric materials include soft, flexible, compliant polymers and blends such as, for example polyethylene (PE)/ethylene vinyl alcohol (EVA) blends, silicone, polyurethane, polyether block amide, thermoplastic elastomers (TPE) and combinations thereof. In another example, the conical echogenic structure 152 is formed from a flexible braided metal mesh, a flexible metal coil, or combinations thereof.

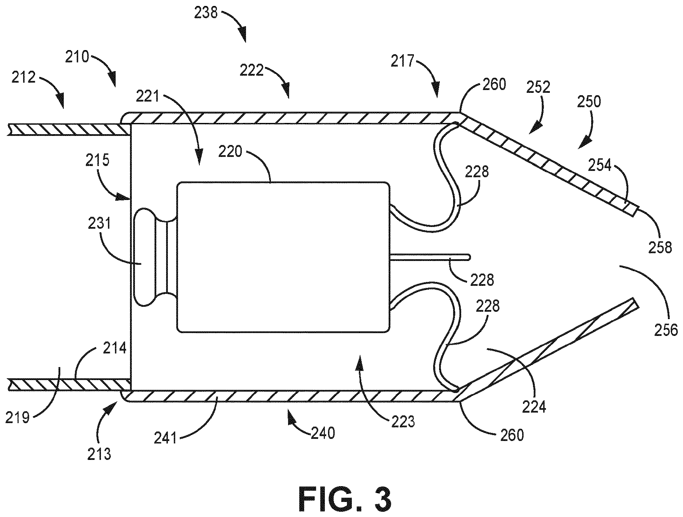

In another example shown schematically in FIG. 3, a distal region 238 of a catheter 212 of a leadless pacemaker deployment system 210 includes a tubular catheter body 214 with an elongate bore 219. As discussed above with reference to FIG. 2, the catheter body 214 can be made of any flexible material, including metals, polymeric materials, and the like.

A distal end 213 of the catheter body 214 includes a delivery cup 222, which includes a generally cylindrical and isodiometric first portion 240 with a wall 241 configured to securely retain a generally cylindrical pacing capsule 220 of a leadless pacemaker as the catheter body 214 is maneuvered through patient vasculature. The first portion 240 of the delivery cup 222 includes a first end 215 that is integral with or connected to the distal end 213 of the tubular body 214 of the catheter 212. A second distal end 217 of the first portion 240 of the delivery cup 222 includes an aperture 224. As discussed above, in various examples the first portion 240 of the delivery cup 222 may be made from a metal, a ceramic material, or a polymeric material that may be the same or different from the polymeric material used to form the tubular body 214 of the catheter 212. In various example examples, the delivery cup 222 may be a portion of the tubular body 214, may be a separate structure press-fit into the tubular body 214, or may be a separate structure bonded to the tubular body 214 by an adhesive, ultrasonic welding, and the like.

The pacing capsule 220 includes a first end 221 retained in the first portion 240 of the delivery cup 222 proximal the first end 215 thereof, and a second distal end 223 proximal the aperture 224. The first end 221 of the pacing capsule 220 includes a tether mount 231, while the second end 223 of the pacing capsule 220 includes an implanting mechanism 226 with an arrangement of tines 228. The implanting mechanism 226 is substantially aligned with the distal end 217 of the first portion 240 of the delivery cup 222 such that the mechanism 226 does not protrude from the aperture 224, and the tines 228 are maintained in an extended state against the wall 241 of the delivery cup 222.

In FIG. 3 the delivery cup 222 includes a second portion 250 that forms a tapered echogenic structure 252 extending away from the distal end 217 of the first portion 240. In the example of FIG. 3, the echogenic structure 252 is a conical projection with a wall 254 that forms a distal tip 258 and a deployment aperture 256 for the pacing capsule 220 and the implanting mechanism 226.

A flexible hinge region 260 is formed between at an interface between the first portion 240 of the delivery cup 222 and the second portion 250 thereof, i.e. at an interface between the wall 254 and the wall 241. In some examples, the hinge region 260 is merely a circumferential area with a reduced wall thickness that allows the wall 254 to flex sufficiently to enlarge the deployment aperture 256 as needed to deploy the pacing capsule 220 into a target tissue, while maintaining the tines 228 in an extended state against the wall 254. In another example, the hinge region 260 is made from a flexible metal or a polymeric material that allows the wall 254 to flex sufficiently to open the deployment aperture 256 as needed for deployment of the pacing capsule 220 while having sufficient structural rigidity to maintain contact between the distal tip 258 and a target tissue such as the heart wall. In some example examples, which are not intended to be limiting, suitable elastomeric polymeric materials for the hinge region 260 include soft, flexible, compliant polymers and blends such as, for example polyethylene (PE)/ethylene vinyl alcohol (EVA) blends, silicone, polyurethane, polyether block amide, thermoplastic elastomers (TPE) and combinations thereof. In another example, the hinge region 260 may be made from a flexible metal braid, a flexible metal coil, and the like.

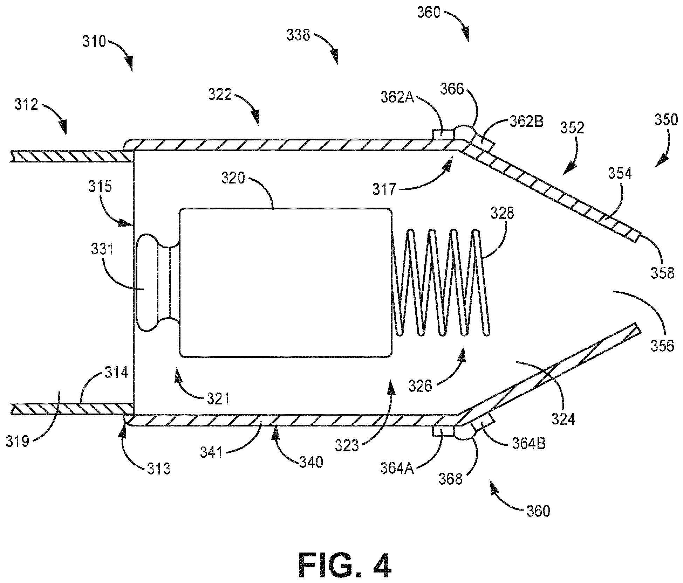

Referring now to FIG. 4, a distal region 338 of a catheter 312 of a leadless pacemaker deployment system 310 includes a tubular catheter body 314 with an elongate bore 319. As discussed above with reference to FIGS. 2-3, the catheter body 314 can be made of any flexible material, including metals, polymeric materials, and the like.

A distal end 313 of the catheter body 214 includes a delivery cup 322, which includes a generally cylindrical and isodiometric first portion 340 with a wall 341. The wall 341 is configured to securely retain a generally cylindrical pacing capsule 320 of a leadless pacemaker as the catheter body 314 is maneuvered through patient vasculature. The first portion 340 of the delivery cup 322 includes a first end 315 that is integral with or connected to the distal end 313 of the tubular body 314 of the catheter 312. A second distal end 317 of the first portion 340 of the delivery cup 322 includes an aperture 324.

As discussed above, in various examples the first portion 340 of the delivery cup 322 may be made from a metal, a ceramic material, or a polymeric material that may be the same or different from the polymeric material used to form the tubular body 314. In various example examples, the delivery cup 322 may be a portion of the tubular body 314, may be a separate structure press-fit into the tubular body 314, or may be a separate structure bonded to the tubular body 314 by an adhesive, ultrasonic welding, and the like.

The pacing capsule 320 includes a first end 321 retained in the first portion 340 of the delivery cup 322 proximal the first end 315 thereof, and a second distal end 323 proximal the aperture 324. The first end of the pacing capsule 320 includes a tether mount 331, while the second end 323 of the pacing capsule 320 includes an implanting mechanism 326 with a helical tip 328. The implanting mechanism 326 is substantially aligned with the distal end 317 of the first portion 340 of the delivery cup 322 such that the implanting mechanism 326 does not protrude from the aperture 324.

In FIG. 4 the delivery cup 322 includes a second portion 350 that forms a tapered echogenic structure 352 extending away from the distal end 317 of the first portion 340. In the example of FIG. 4, the conical echogenic structure 352 includes a wall 354that forms a distal tip 358 and a deployment aperture 356 for the pacing capsule 320.

A flexible hinge region 360 is formed between at an interface between the first portion 340 of the delivery cup 322 and the second portion 350 thereof, i.e. at an interface between the wall 354 and the wall 341. In the example of FIG. 4, the hinge region 360 includes a mechanical hinge construction. In the example of FIG. 4, which is not intended to be limiting, the mechanical hinge construction includes leaves 362A, 364A on the wall 341, as well as leaves 362B, 364B on the wall 354. The leaves 362A-B and 364A-B may be formed integrally with the wall 341, 354 by a technique such as molding, 3D printing, additive manufacturing and the like, or may be bonded to the respective walls by an adhesive, ultrasonic welding, mechanical fasteners, and combinations thereof.

The hinge regions 360 further include a first arcuate spring-like connector 366 between the leaves 362A, 362B and a second arcuate spring-like connector 368 between the leaves 364A, 364B. The spring-like connectors 366, 368, which may be the same or different, maintain the position of the wall 354 until the pacing capsule 320 is deployed, then flex sufficiently to enlarge the deployment aperture 356 as needed so the pacing capsule 320 can pass therethrough such that the helix tip 328 can be anchored into a target tissue such as the heart wall. The spring-like connectors 366, 368 should also be sufficiently rigid such that the distal tip 358 maintains contact with the target tissue and the conical echogenic structure 352 does not substantially deform. In various examples, the connectors 366, 368 may be formed integrally with the leaves 362A-B and 364A-B by molding, 3D printing, additive manufacturing and the like, or may be bonded to the leaves 362A-B and 364A-B with an adhesive, ultrasonic welding, mechanical fasteners, and combinations thereof.

In some example examples, which are not intended to be limiting, suitable elastomeric polymeric materials for the connectors 366, 368 include flexible, compliant polymers and blends such as, for example polyethylene (PE)/ethylene vinyl alcohol (EVA) blends, silicone, polyurethane, polyether block amide, thermoplastic elastomers (TPE) and combinations thereof. In another example, the connectors 366, 368 made from a flexible metal braid, a flexible metal wire, and the like.

The hinge construction shown in FIG. 4 is not intended to be limiting, and suitable hinge constructions may vary widely in both design and materials selection. For example, the leaves 362A-B and 364A-B can be made of a metal riveted to the walls 341, 354, or may be attached with screws or other types of fasteners. The connectors 366, 368 can optionally include pins and other reinforcing structures as necessary to maintain the orientation of the wall 354 or may optionally including springs and other elements to modify the resistance to opening of the wall 354.

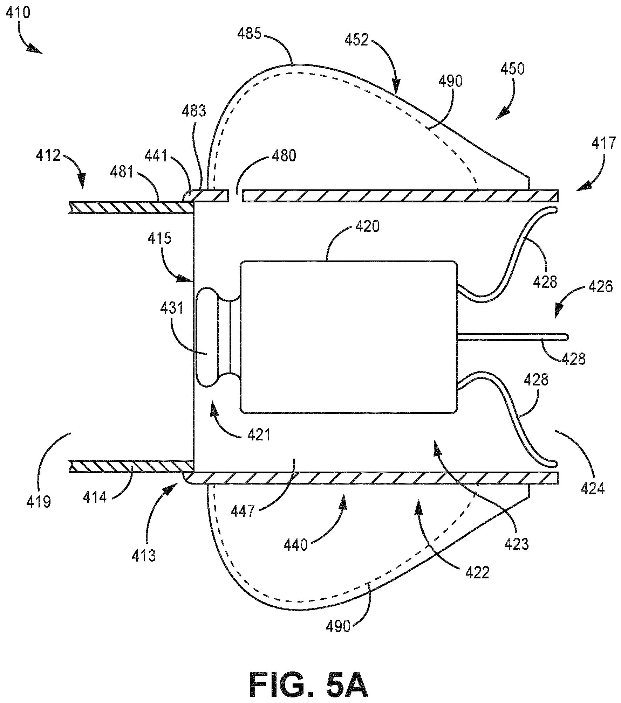

Referring now to FIG. 5A, in another example a distal region 438 of a catheter 412 of a leadless pacemaker deployment system 410 includes a tubular catheter body 414 with an elongate bore 419. As discussed above with reference to FIGS. 2-4, the catheter body 414 can be made of any flexible material, including metals, polymeric materials, and the like.

A distal end 413 of the catheter body 414 includes a delivery cup 422, which includes a generally cylindrical and isodiometric first portion 440 with a wall 441. The wall 441 is configured to securely retain in a bore 447 a generally cylindrical pacing capsule 420 of a leadless pacemaker as the catheter body 414 is maneuvered through patient vasculature. The first portion 440 of the delivery cup 422 includes a first end 415 that is integral with or connected to the distal end 413 of the tubular body 414 of the catheter 412. A second distal end 417 of the first portion 440 of the delivery cup 422 includes an aperture 424. In some examples (not shown in FIG. 5A), the distal end 417 may include an optional tapering dilating tip.

As discussed above, in various examples the first portion 440 of the delivery cup 422 may be made from a metal, a ceramic material, or a polymeric material that may be the same or different from the polymeric material used to form the tubular body 414. In various example examples, the delivery cup 422 may be a portion of the tubular body 414, may be a separate structure press-fit into the tubular body 414, or may be a separate structure bonded to the tubular body 414 by an adhesive, ultrasonic welding, and the like.

The pacing capsule 420 includes a first end 421 retained in the first portion 440 of the delivery cup 422 proximal the first end 415 thereof, and a second distal end 423 proximal the aperture 424. The first end 421 of the pacing capsule 420 includes a tether anchor 431, and the second end 423 of the pacing capsule 420 includes an implanting mechanism 426 with an arrangement of tines 428. The implanting mechanism 426 is substantially aligned with the distal end 417 of the first portion 440 of the delivery cup 422 such that the mechanism 426 does not protrude from the aperture 424.

In FIG. 5A the delivery cup 422 includes a second portion 450 with at least one echogenic compliant balloon 452. In FIG. 5A, the echogenic balloon 452 is shown in an inflated state, and in some examples, which are not intended to be limiting, has a conical or pear-like shape, but balloons with a wide variety of shapes can be used.

The echogenic balloon 452 may be attached to an external surface 481 of a wall of the tubular body 414 of the catheter 412, to an external surface 483 of the wall 441 of the first portion 440 of the delivery cup 422, or a combination thereof. In some example examples, which are not intended to be limiting, the balloon 452 is formed from a soft, flexible, compliant polymeric material such as, for example polyethylene (PE)/ethylene vinyl alcohol (EVA) blends, silicone, polyurethane, polyether block amide, and combinations thereof. The balloon 452 includes a balloon wall 485 that may be formed from a single layer or multiple layers of polymeric materials, and may optionally include reinforcing materials to enhance strength and burst resistance. In some example examples, the balloon 452 has a length of about 2 cm to about 10 cm. The balloon wall 485 can be attached to the external surfaces 481, 483 by any suitable technique including, for example, bonding, fusing, adhesives, and the like. The echogenicity of the balloon 452 can be enhanced by the same methods as described in the examples above.

In various examples, the echogenic balloon 452 may extend around the full circumference of the tubular catheter body 414 or the delivery cup 422. In another example, the balloon 452 extends only a portion of the way around the circumference of the tubular catheter body 414 or the delivery cup 422.

In operation, to improve contrast on a sonogram with a particular patient tissue, a fluid is introduced into the fluid port 36 (FIG. 1A), travels down the catheter bore 419, and exits the fluid egress port 480 to inflate and expand the balloon 452.

In various examples, which are not intended to be limiting, the balloon 452 is inflated with an ultrasonically transparent fluid such as water or saline, a non-ultrasonically transparent fluid such as a radio-opaque contrast medium, or a mixture or combination thereof, via the fluid egress port 480 after the device is introduced to the body/heart to provide shape to the device and therefore orientation feedback regarding the position of the distal end 417 of the delivery cup 422 on a sonogram as described in the examples above. For example, when viewed in a sonogram, the conical balloon 452 would be expected to provide an image generally following the dashed line 490. The use of a balloon could be used to provide a more echogenic shape to the delivery cup 422 without a required increase in the length of the delivery cup 422. The thin, uninflated balloon 452 minimally increases the diameter of the device 410 during portions of the procedure where minimal diameter is important (for example, to provide improved venous access).

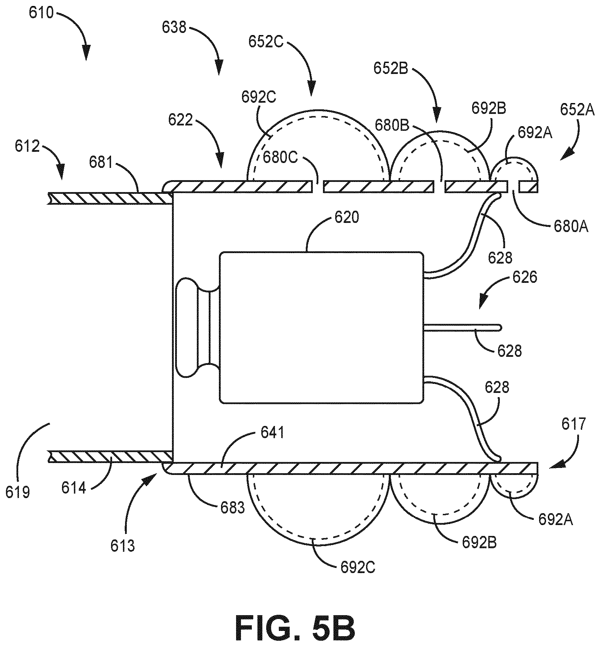

Referring now to FIG. 5B, in another example a distal region 638 of a catheter 612 of a leadless pacemaker deployment system 610 includes a tubular catheter body 614 with an elongate bore 619. A distal end 613 of the catheter body 614 includes a delivery cup 622 with a wall 641 configured to securely retain a generally cylindrical pacing capsule 620 of a leadless pacemaker. The pacing capsule 620 includes an implanting mechanism 626 with an arrangement of tines 628.

In FIG. 5B the delivery cup 622 includes a plurality of echogenic compliant balloons 652A, 652B, 652C. The echogenic balloons 652A-C are shown in an inflated state, and in some examples, which are not intended to be limiting, have a generally spherical shape, but balloons with a wide variety of shapes can be used.

The echogenic balloons 652A-C may be attached to an external surface 681 of a wall of the tubular body 614 of the catheter 612, to an external surface 683 of the wall 641 of the delivery cup 622, or a combination thereof. The balloons 652A-C may be formed from the same types of flexible polymeric materials described above, and may include walls formed from a single layer or multiple layers of polymeric materials. The balloons 652A-C may be attached to the external surfaces 681, 683 by any suitable technique including, for example, bonding, fusing, adhesives, and the like.

In various examples, which are not intended to be limiting, the balloons 652A-652C can be inflated with an ultrasonically transparent fluid such as water or saline, a non-ultrasonically transparent fluid such as a radio-opaque contrast medium, or a mixture or combination thereof, via the respective fluid egress ports 680A-680C after the device is introduced to the body/heart to provide shape to the device and therefore orientation feedback regarding the position of the distal end 617 of the delivery cup 622 on a sonogram as described in the examples above. For example, when viewed in a sonogram, the spherical balloons 652A-C would be expected to provide an image generally following the dashed lines 692A-C. The use of the balloons 652A-C provides a more echogenic shape to the delivery cup 622 without a required increase in the length of the delivery cup 622. The thin, uninflated balloon 652A-C minimally increases the diameter of the device 610 during portions of the procedure where minimal diameter is important (for example, to provide improved venous access).

In some examples, not shown in FIGS. 5A-5B, the echogenic balloons 452 and 652A-C may be employed in combination with the tapering echogenic structures shown in FIGS. 2-4 above.

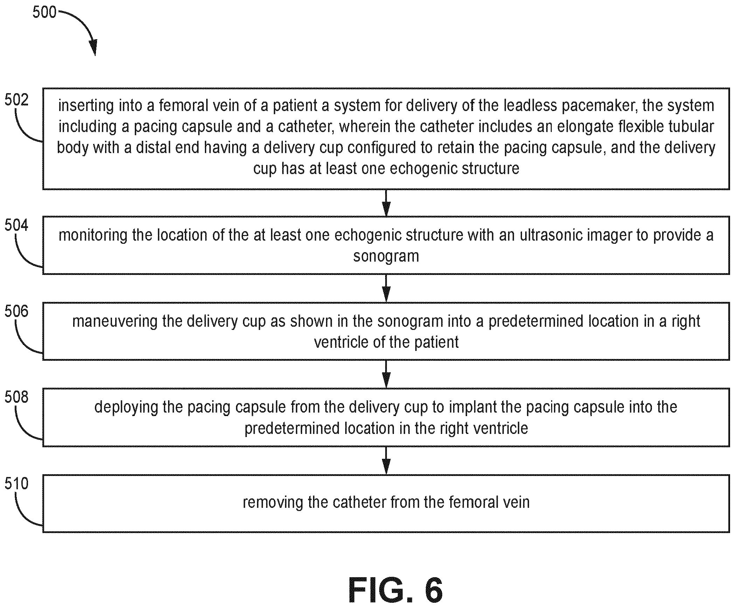

In another example shown in the flow chart of FIG. 6, the present disclosure is directed to a method 500 for implanting a leadless pacemaker in a target tissue.

The example method includes inserting into a femoral vein of a patient a system for delivery of the leadless pacemaker (502). The system includes a pacing capsule and a catheter. The catheter includes an elongate flexible tubular body with a distal end having a delivery cup configured to retain the pacing capsule, and the delivery cup comprises at least one echogenic structure as described in FIGS. 2-5 above.

The example method includes monitoring the location of the at least one echogenic structure with an ultrasonic imager to provide a sonogram (504). Any suitable ultrasonic imaging system may be used, and in some examples the imaging system includes an ultrasonic probe that moves along an external surface of the skin of the patient. In another example, an intracardiac echo (ICE) probe may be inserted into the esophagus or nasal passages of the patient and maneuvered into position in the esophagus to image the anatomy of the patient. In various examples, which are not intended to be limiting, suitable probe apparatus include ultrasonic probes available from General Electric (GE), Philips, Siemens and the like.

In various examples, which are provided by way of example and are not intended to be limiting, the transducers in the ultrasonic probe apparatus operate over a frequency range of about 1 MHz to about 60 MHz, or about 3 MHz to about 10 MHz for imaging procedures, and have a focal length of about 1 cm to about 4 cm, or about 2 cm to about 3 cm.

The example method includes maneuvering the delivery cup as shown in the sonogram into a predetermined location in a heart of the patient (506).

The example method includes deploying the pacing capsule from the delivery cup to implant the pacing capsule into the predetermined location in the heart (508).

The example method includes removing the catheter from the femoral vein (510).

Various examples have been described.

## Claims

1. A catheter for delivery of a leadless pacemaker, the catheter comprising an elongate flexible tubular body (114) with a proximal end (111) and a distal end (113), wherein the distal end of the tubular body comprises a delivery cup (122) configured to releasably retain a pacing capsule (120) of the leadless pacemaker, and wherein the delivery cup comprises an echogenic structure, wherein the delivery cup comprises a first portion (140) with a first diameter, and a second portion (150) comprising a tapering conical tip with a distal aperture (124), wherein the distal aperture has a second diameter less than the first diameter.

2. The catheter of claim 1, further comprising a hinge (260) at an interface between the first portion of the delivery cup and the second portion of the delivery cup.

3. The catheter of any one of claims 1 to 2, wherein the second portion of the delivery cup comprises a compliant balloon (452) overlying the first portion of the delivery cup, and wherein the balloon is attached to an external surface of the first portion thereof.

4. The catheter of claim 4, wherein the first portion of the delivery cup comprises a fluid egress port (480) fluidly connected to the balloon.

5. The catheter of claim 4 or 5, wherein the balloon extends around a circumference of the external surface of the first portion of the delivery cup.

6. The catheter of claim 4 or 5, wherein the balloon extends around a portion of a circumference of the external surface of the tubular body, and wherein the portion of the circumference is less than the entire circumference.

7. The catheter of any one of claims 4 to 7, wherein the catheter comprises a plurality of balloons.

8. The catheter of any one of claims 4 to 8, wherein the balloon, when inflated with a fluid, has a conical shape.

9. A system for delivery of a leadless pacemaker, the system comprising:
a pacing capsule (120); and
a catheter (112) of any one of claim 1 to 10.

10. The system of claim 10, wherein the delivery cup comprises:
a first portion (140) configured to releasably retain the pacing capsule, and
a second portion (150) distal to the first portion, wherein the second portion comprises the echogenic structure.

11. The system of claim 11, wherein the echogenic structure on the second portion of the delivery cup comprises a conical protrusion (154) with a distal aperture (124).

12. The system of claim 11 or 12, further comprising a hinge (260) at an interface between the first portion of the delivery cup and the second portion of the delivery cup.

13. The system of any one of claims 10 to 13, wherein the echogenic feature comprises at least one balloon (452) on an external surface of the delivery cup.

## Patentansprüche

1. Katheter zum Einführen eines kabellosen Herzschrittmachers, der Katheter umfassend einen länglichen, flexiblen, röhrenförmigen Körper (114) mit einem proximalen Ende (111) und einem distalen Ende (113), wobei das distale Ende des röhrenförmigen Körpers eine Einführungsschale (122) umfasst, die konfiguriert ist, um eine Stimulationskapsel (120) des kabellosen Herzschrittmachers lösbar zu halten, und wobei die Einführungsschale eine echogene Struktur umfasst, wobei die Einführungsschale einen ersten Abschnitt (140) mit einem ersten Durchmesser und einen zweiten Abschnitt (150) umfasst, umfassend eine sich verjüngende, konische Spitze mit einer distalen Öffnung (124), wobei die distale Öffnung einen zweiten Durchmesser aufweist, der kleiner als der erste Durchmesser ist.

2. Katheter nach Anspruch 1, ferner umfassend ein Scharnier (260) an einer Schnittstelle zwischen dem ersten Abschnitt der Einführungsschale und dem zweiten Abschnitt der Einführungsschale.

3. Katheter nach einem der Ansprüche 1 bis 2, wobei der zweite Abschnitt der Einführungsschale einen nachgiebigen Ballon (452) umfasst, der über dem ersten Abschnitt der Einführungsschale liegt, und wobei der Ballon an einer Außenoberfläche des ersten Abschnitts angebracht ist.

4. Katheter nach Anspruch 4, wobei der erste Abschnitt der Einführungsschale einen Fluidaustrittsanschluss (480) umfasst, der mit dem Ballon fluidisch verbunden ist.

5. Katheter nach Anspruch 4 oder 5, wobei sich der Ballon um einen Umfang der Außenoberfläche des ersten Abschnitts der Einführungsschale erstreckt.

6. Katheter nach Anspruch 4 oder 5, wobei sich der Ballon um einen Abschnitt eines Umfangs der Außenoberfläche des röhrenförmigen Körpers erstreckt und wobei der Abschnitt des Umfangs kleiner als der Gesamtumfang ist.

7. Katheter nach einem der Ansprüche 4 bis 7, wobei der Katheter eine Vielzahl von Ballons umfasst.

8. Katheter nach einem der Ansprüche 4 bis 8, wobei der Ballon, wenn er mit einem Fluid aufgeblasen ist, eine konische Form aufweist.

9. System für die Einführung eines kabellosen Herzschrittmachers, das System umfassend:
eine Stimulationskapsel (120); und
einen Katheter (112) nach einem der Ansprüche 1 bis 10.

10. System nach Anspruch 10, wobei die Einführungsschale umfasst:
einen ersten Abschnitt (140), der konfiguriert ist, um die Stimulationskapsel lösbar zu halten, und
einen zweiten Abschnitt (150) distal zu dem ersten Abschnitt, wobei der zweite Abschnitt die echogene Struktur umfasst.

11. System nach Anspruch 11, wobei die echogene Struktur auf dem zweiten Abschnitt der Einführungsschale einen konischen Vorsprung (154) mit einer distalen Öffnung (124) umfasst.

12. System nach Anspruch 11 oder 12, ferner umfassend ein Scharnier (260) an einer Schnittstelle zwischen dem ersten Abschnitt der Einführungsschale und dem zweiten Abschnitt der Einführungsschale.

13. System nach einem der Ansprüche 10 bis 13, wobei das echogene Merkmal mindestens einen Ballon (452) auf einer Außenoberfläche der Einführungsschale umfasst.

## Revendications

1. Cathéter pour l'administration d'un stimulateur cardiaque sans sonde, le cathéter comprenant un corps tubulaire flexible allongé (114) avec une extrémité proximale (111) et une extrémité distale (113), dans lequel l'extrémité distale du corps tubulaire comprend une cupule d'administration (122) conçue pour retenir de manière amovible une capsule de stimulation (120) du stimulateur cardiaque sans sonde, et dans lequel la cupule d'administration comprend une structure échogène, dans lequel la cupule d'administration comprend une première partie (140) avec un premier diamètre, et une seconde partie (150) comprenant une pointe conique effilée avec une ouverture distale (124), dans lequel l'ouverture distale a un second diamètre inférieur au premier diamètre.

2. Cathéter selon la revendication 1, comprenant en outre une charnière (260) au niveau d'une interface entre la première partie de la cupule d'administration et la seconde partie de la cupule d'administration.

3. Cathéter selon l'une quelconque des revendications 1 à 2, dans lequel la seconde partie de la cupule d'administration comprend un ballonnet souple (452) recouvrant la première partie de la cupule d'administration, et dans lequel le ballonnet est fixé à une surface externe de la première partie de celle-ci.

4. Cathéter selon la revendication 4, dans lequel la première partie de la cupule d'administration comprend un orifice de sortie de fluide (480) relié de manière fluidique au ballonnet.

5. Cathéter selon la revendication 4 ou 5, dans lequel le ballonnet s'étend autour d'une circonférence de la surface externe de la première partie de la cupule d'administration.

6. Cathéter selon la revendication 4 ou 5, dans lequel le ballonnet s'étend autour d'une partie d'une circonférence de la surface externe du corps tubulaire, et dans lequel la partie de la circonférence est inférieure à la totalité de la circonférence.

7. Cathéter selon l'une quelconque des revendications 4 à 7, dans lequel le cathéter comprend une pluralité de ballonnets.

8. Cathéter selon l'une quelconque des revendications 4 à 8, dans lequel le ballonnet, lorsqu'il est gonflé avec un fluide, a une forme conique.

9. Système d'administration d'un stimulateur cardiaque sans sonde, comprenant :
une capsule de stimulation (120) ; et
un cathéter (112) selon l'une quelconque revendication 1 à 10.

10. Système selon la revendication 10, dans lequel la cupule d'administration comprend :
une première partie (140) conçue pour retenir de manière amovible la capsule de stimulation, et
une seconde partie (150) distale par rapport à la première partie, dans lequel la seconde partie comprend la structure échogène.

11. Système selon la revendication 11, dans lequel la structure échogène sur la seconde partie de la cupule d'administration comprend une saillie conique (154) avec une ouverture distale (124).

12. Système selon la revendication 11 ou 12, comprenant en outre une charnière (260) au niveau d'une interface entre la première partie de la cupule d'administration et la seconde partie de la cupule d'administration.

13. Système selon l'une quelconque des revendications 10 à 13, dans lequel la caractéristique échogène comprend au moins un ballonnet (452) sur une surface externe de la cupule d'administration.
